# EUROPÄISCHE PATENTSCHRIFT

(11) **EP 2 125 085 B1**
(45) Veröffentlichungstag und Bekanntmachung des Hinweises auf die Patenterteilung: **30.05.2012**
(21) Anmeldenummer: 08700846.2
(22) Anmeldetag: 07.01.2008
(51) Int. Cl.: A61M 5/32

(54) **VORRICHTUNG ZUR INJEKTION VON FLÜSSIGKEITEN**
DEVICE FOR INJECTING LIQUIDS
DISPOSITIF POUR L'INJECTION DE LIQUIDES

(30) Priorität: 09.01.2007 DE 102007002096
(43) Veröffentlichungstag der Anmeldung: 02.12.2009
(73) Patentinhaber: B. Braun Melsungen AG, 34212 Melsungen (DE)
(72) Erfinder: HARTMANN, Michael, 34212 Melsungen (DE)
(74) Vertreter: Arth, Hans-Lothar
(86) Internationale Anmeldenummer: PCT/DE2008/000010
(87) Internationale Veröffentlichungsnummer: WO 2008/083664

(56) Entgegenhaltungen:
- WO-A-2004/047894
- WO-A-2005/079889
- US-A- 5 695 477
- US-A1- 2003 014 018

## Beschreibung

Die Erfindung betrifft eine Vorrichtung zur Injektion von Flüssigkeiten, insbesondere von flüssigen Medikamenten beziehungsweise Hormonpräparaten wie beispielsweise Insulin, nach dem Oberbegriff des Anspruchs 1.

### Stand der Technik

Eine solche Vorrichtung wird beispielsweise in der EP 0 554 995 B1 beschrieben. Bei der dortigen als Injektionsstift ausgebildeten Vorrichtung ist die Kanülenaufnahme mit einem Außengewinde versehen, auf welches der ein Innengewinde aufweisende und eine Kanüle tragende Kanülenhalter aufschraubbar ist. Vorrichtungen dieser Art werden häufig von Personen benutzt, welche sich bestimmte Medikamente, wie beispielsweise Insulin, selbst verabreichen. Die dabei verwendeten Kanülen, die aus hygienischen Gründen und der damit verbundenen Infektionsgefahr nur einmal verwendet werden sollen, müssen nach der Injektion des Medikamentes entsorgt und sicher aufbewahrt werden.

Um den die Kanüle tragenden Kanülenhalter von der Kanülenaufnahme zu lösen, muss der Kanülenhalter von der Kanülenaufnahme abgeschraubt werden. Dabei muss der Kanülenhalter direkt mit den Fingern angefasst werden und es besteht die Gefahr, sich beim Abschrauben des Kanülenhalters an der frei liegenden Kanülenspitze zu verletzen.

Auch wenn die Kanülenspitze mit einer Kappe versehen werden kann, wie dies beispielsweise in der EP 0 688 571 B1 gezeigt ist, ist die Verletzungsgefahr stets vakant, da beim Aufsetzen der Kappe die Kanülenspitze freiliegt.

Insbesondere bei Personen, welche sich die Medikamente üblicherweise in der eigenen Wohnung oder auch in öffentlichen Gebäuden selbst injizieren, sind auch weiter Personen den inhärenten Gefahren benutzter Kanülen ausgesetzt, insbesondere dann, wenn sie dem Patienten das Medikament injizieren oder bei der Injektion beziehungsweise beim Kanülenwechsel dem Patienten unterstützend behilflich sind. Damit beim Lösen des Kanülenhalters von der Kanülenaufnahme die Verletzungs- und damit auch die Infektionsgefahr minimiert ist, wurden Entsorgungsbehälter vorgeschlagen, in welche ein spezielles Werkzeug zum Lösen des Kanülenhalters integriert ist und worin die benutzen Kanülen sicher aufbewahrt werden können. Die sichere Entsorgung von Kanülen wird sowohl von den die Vorrichtung benutzenden Personen als auch von der Gesetzgebung in den meisten Ländern gefordert.

Ein solcher Entsorgungsbehälter mit integriertem Lösewerkzeug für den Kanülenhalter ist beispielsweise in der EP 1 396 234 B1 beschrieben. Zum Lösen des Kanülenhalters von der Kanülenaufnahme ist dabei der Entsorgungsbehälter mit einer Öffnung versehen, die eins spezielle, an den Kanülenhalter angepasste Geometrie aufweist. Der Kanülenhalter wird zum Lösen von der Kanülenaufnahme in diese Öffnung gesteckt und der Injektionsstift gedreht, wobei sich der Kanülenhalter aufgrund der korrespondierenden Gewinde nun von der Kanülenaufnahme löst. Auch wenn die in dieser Schrift vorgeschlagene Lösung ein Festklemmen des Kanülenhalters in der Öffnung verhindern soll, kann ein Festklemmen nicht gänzlich ausgeschlossen werden, wenn die aufeinander abgestimmten Geometrien des Kanülenhalters und der Öffnung Fehlertoleranzen aufweisen. Der so in der Öffnung festsitzenden Kanülenhalter muss in einem zweiten Arbeitsschritt durch die Öffnung hindurchgedrückt werden. Dies kann mit einem weiteren Werkzeug geschehen. Es kann aber auch durch den Benutzer erfolgen, indem er versucht den Kanülenhalter mit seinen Fingern durch die Öffnung in den Entsorgungsbehälter zu drücken. Dabei ist er natürlich wieder der inhärenten Gefahr einer Verletzung beziehungsweise Infektion ausgesetzt, da aus dem Kanülenhalter ein Ende der Kanüle herausragt und somit freiliegt. Dieses Ende der Kanülen ist auch nicht mit einer Kappe oder ähnlichem abgedeckt, da es bis zum Lösen des Kanülenhalters in der Kartusche positioniert war, um ein Injizieren des Medikamentes zu ermöglichen.

Die in der EP 1 396 234 B1 beschriebenen Öffnungen sind auch nicht in der Lage, Kanülenhalter von Kanülenaufnahmen zu lösen, wenn die Geometrie der Öffnung nicht an die Geometrie des Kanülenhalters angepasst ist.

US 5965477 beschreibt ein Sicherheitssystem zur Vermeidung von Stichverletzungen mit Injektionsnadeln für die Verwendung von injektionsspritzenartigen Kanülen, das einen spritzenartigen Hohlkörper, eine Führvorrichtung zur linearen Bewegung einer Nadel und ein Sammelbehältnis für eine kontrollierte Abgabe von gebrauchten Nadeln umfasst, wobei der spritzenartigen Hohlkörper wiederverwendet werden kann.

### Aufgabenstellung

Es ist daher Aufgabe der Erfindung, eine Vorrichtung nach dem Oberbegriff des Anspruchs 1 bereitzustellen, die ein einfaches und gefahrenfreies Lösen des Kanülenhalters von der Kanülenaufnahme ermöglicht, wobei auf ein separates, an die Geometrie des Kanülenhalters angepasstes Werkzeug, welches gegebenenfalls an einem Entsorgungsbehälter angeordnet ist, verzichtet werden kann.

### Erfindung und vorteilhafte Wirkungen

Gelöst wird diese Aufgabe durch eine Vorrichtung mit den Merkmalen des Anspruchs 1. Vorteilhafte Ausgestaltungen der Erfindung finden sich in den Unteransprüchen.

Dadurch, dass nach der Erfindung zum Lösen des Kanülenhalters ein an dem Kanülenhalter angreifendes und durch ein Betätigungselement aktivierbares Löseelement vorgesehen ist, wird der Benutzer der Vorrichtung unabhängig von einem separaten Werkzeug zum Lösen des Kanülenhalters. Zum Entsorgen einer benutzten Kanüle ist nur noch die erfindungsgemäße Vorrichtung und ein Entsorgungsbehälter notwendig. Dabei muss die Geometrie des Kanülenhalters nicht an die Geometrie eines separaten, gegebenenfalls an dem Entsorgungsbehälter angeordneten Werkzeuges angepasst sein. Der Benutzer der erfindungemäßen Vorrichtung wird somit unabhängig von bestimmten, auf die Vorrichtung abgestimmten Entsorgungsbehälter beziehungsweise Werkzeuge, da der Mechanismus zum Lösen des Kanülenhalters Bestandteil der Vorrichtung selbst ist. Dadurch ist natürlich auch die Lebensqualität der Personen, welche auf solche Vorrichtungen zum selbständigen Injizieren von Medikamenten angewiesen sind, deutlich erhöht, da separate Lösewerkzeuge beziehungsweise spezielle Entsorgungsbehälter nicht mehr notwendig sind und somit nicht mit mitgeführt werden müssen.

Nach einer ersten vorteilhaften Ausgestaltung der Erfindung ist die Vorrichtung zum manuellen Lösen des Kanülenhalters ausgebildet. Durch diese Maßnahme ist eine kostengünstig und einfache Vorrichtung gegeben, vermittels welcher der Kanülenhalter mit Hilfe einer manuell betätigbaren Mechanik von der Kanülenaufnahme gelöst werden kann.

Alternativ ist es natürlich möglich, dass die Vorrichtung zum motorischen, insbesondere elektromotorischen Lösen des Kanülenhalters ausgebildet ist. Dies Ausgestaltung der Erfindung ist für den Benutzer besonders komfortabel, da nur das Betätigungselement bedient werden muss, um das Lösen des Kanülenhalters von der Kanülenaufnahme zu aktivieren. Die manuelle Bedienung einer Mechanik durch den Benutzer ist nicht notwendig, da dies motorisch beziehungsweise elektromotorisch geschieht.

Als besonders benutzerfreundlich hat sich herausgestellt, wenn das Gehäuseteil im Wesentlichen stiftförmig beziehungsweise axialsymmetrisch bezüglich seiner Längsachse ausgebildet ist. Somit ist eine bequeme und komfortable Bedienung der Vorrichtung möglich, die sie sich in ergonomischer Weise der Hand des Benutzers anpasst.

Dabei hat sich die Ausgestaltung der Vorrichtung als Injektionsstift besonders bewährt, da ein solcher Injektionsstift bequem und komfortabel mit einer Hand bedient werden kann.

Erfindungsgemäß ist das Löseelement in axialer Richtung an oder auf dem Gehäuseteil verschiebbar angeordnet. Durch diese Maßnahme ist beim Lösen des Kanülenhalters kein weiterer Platzbedarf in radialer Richtung der erfindungsgemäßen Vorrichtung notwendig. Die Vorrichtung kann dann beim Lösen des Kanülenhalters von der Kanülenaufnahme bereits so an einem Entsorgungsbehälter positioniert werden, dass der Kanülenhalter nach dem Lösen nur in den Entsorgungsbehälter entweichen kann.

Vorteilhafterweise sind das Löseelement und das Betätigungselement starr, insbesondere stoffschlüssig miteinander verbunden, wodurch auf eine aufwendige Mechanik zwischen Betätigungs- und Löseelement verzichtet werden kann.

Alternativ ist es auch möglich, insbesondere bei einem motorisch beziehungsweise elektromotorisch angetrieben Löseelement, dass das Löseelement und das Betätigungselement nicht starr, sondern über Getriebemittel miteinander verbunden sind. Eine solche Ausführungsform erfordert allerdings eine aufwendigere Mechanik, damit ein Lösen des Kanülenhalters von der Kanülenaufnahme möglich ist.

Das Löseelement und/oder das Betätigungselement sind entlang einer Führung an oder auf dem Gehäuseteil verschiebbar angeordnet. Dabei ist die Führung als wenigstens eine lineare Führungsschiene in axialer Richtung des Gehäuseteils ausgebildet, wobei an das Löseelement und/oder das Betätigungselement Elemente zum Eingriff in die wenigstens eine Führungsschiene angeordnet sind. Hierdurch wird ein durch einfaches Verschieben des Löseelementes und/oder des Betätigungselementes ein Lösen des Kanülenträgers von der Kanülenaufnahme bewirkt.

Die Führung kann aber auch als ein auf das Gehäuseteil angeordnetes Außengewinde ausgebildet sein, welches mit einem Innengewinde des Löseelementes und/oder des Betätigungselementes zusammenwirkt.

In beiden Fällen kann es jedoch vorgesehen sein, dass als Löseelement und/oder als Betätigungselement eine auf dem Gehäuseteil axial verschiebbare Hülse vorgesehen ist. Durch eine solche Hülse wird die Dimensionierung der Vorrichtung im Wesentlichen nicht beeinträchtigt, so dass weiterhin eine bequeme und komfortable Handhabung der erfindungsgemäßen Vorrichtung möglich ist.

Nach einer weiteren vorteilhaften Ausgestaltung der Erfindung kontaktiert das Löseelement zum Lösen des Kanülenhalters diesen form- und/oder reibschlüssig und überträgt seine axiale Bewegung auf den Kanülenhalter, wodurch dieser ebenfalls in axialer Richtung des Gehäuseteils verschoben wird, bis er sich von der Kanülenaufnahme gelöst hat. Es sind keine weiteren Teile notwendig, um das Lösen des Kanülenhalters durchzuführen. Die Vorrichtung ist somit auch hinsichtlich der eingesetzten Teile optimiert.

Das Löseelement kann dabei eine ringförmige Begrenzung aufweisen, die in Gebrauchsstellung den auf der Kanülenaufnahme angeordneten Kanülenhalter hintergreift. Alternativ ist es natürlich auch möglich, dass an dem Löseelement Vorsprünge angeordnet sind, welche beim Lösen des Kanülenhalters von der Kanülenaufnahme in korrespondierende Aufnahmen des Kanülenhalters eingreifen. In beiden Fällen ist ein sicheres und einfaches Lösen des Kanülenhalters gewährleistet.

Um ein unbeabsichtigtes Lösen des Kanülenhalters vom Kartuschenhalter zu verhindern, ist es vorgesehen, dass das Löseelement und/oder das Betätigungselement durch wenigstens ein Fixierungselement fixierbar sind/ist.

Vorteilhafterweise ist nach einer weiteren Ausgestaltung der Erfindung das Löseelement beim Aufsetzen eines neuen, unbenutzten Kanülenhalters durch diesen wieder in seine Ausgangsposition überführbar. In einer besonders komfortablen Ausgestaltung ist das Löseelement allerdings mit einem Rückholmechanismus versehen - beispielsweise durch Federkraftbeaufschlagung - wodurch das Löseelement nach dem Lösen des Kanülenhalters von der Kanülenaufnahme selbsttätig in sein Ausgangsposition überführt wird.

Das Gehäuseteil ist in einer Ausgestaltung der Erfindung als Kartuschenhalter ausgebildet. Dabei kann das Gehäuseteil auch eine Dosiereinrichtung aufweisen. Der Kartuschenhalter und die Dosiereinrichtung können nach einer weiteren Ausgestaltung der Erfindung mittels einer Kupplung lösbar miteinander verbindbar sein.

Um den Füllstand einer aus transparentem Material bestehenden Kartusche zu kontrollieren hat es sich als vorteilhaft herausgestellt das Gehäuseteil und/oder den Kartuschenhalter und/oder die Hülse zumindest teilweise transparent auszugestalten oder mit einer Aussparung, beispielsweise in Form eines Sichtfensters zu versehen.

Von Vorteil ist auch, wenn das Gehäuseteil oder die Kartusche Kupplungsmittel aufweisen, welche mit entsprechenden Gegenkupplungsmittel des Kanülenhalters korrespondieren. Durch diese Maßnahme ist gewährleistet, dass das Gehäuseteil beziehungsweise die Kartusche fest mit dem Kanülenhalter verbunden werden kann. Ein Lösen des Kanülenhalters von der erfindungsgemäßen Vorrichtung ist somit während der Applikation beziehungsweise des Injizieren des Medikamentes wirkungsvoll vorgebeugt.

Dabei hat es sich als besonders vorteilhaft erwiesen, dass die Kupplungsmittel als Federelemente und die Gegenkupplungsmittel damit korrespondierende Aussparungen, in welche die Federelemente eingreifen, ausgebildet sind. natürlich können die Federelemente auch an Haftreibungsflächen ausgebildet sein, an welchen die Federelemente zu liegen kommen, wobei über die Haftreibung ein sichere Sitz des Kanülenhalters auf der Kanülenaufnahme während der Injektion sichergestellt ist.

Alternativ können die Kupplungsmittel und die Gegenkupplungsmittel als Rast- und Gegenrastelemente beziehungsweise als Clips- und Gegenclipselemente ausgebildet sind.

Nach einer weiteren Ausgestaltung der Erfindung sind die Kupplungsmittel und die Gegenkupplungsmittel als Außen- und Innengewinde ausgebildet. In allen Fällen ist durch dies Ausgestaltungen ein sicherer Sitz des Kanülenhalters auf der Kanülenaufnahme während der Applikation beziehungsweise des Injizieren des Medikamente gewährleistet, wobei danach ein einfaches und gefahrloses Lösen des Kanülenhalters von der Kanülenaufnahme möglich ist

Um die gesamte Vorrichtung aus hygienischen Gründen vor einer Verschmutzung zu schützen, ist weiterhin eine Schutzkappe für das Gehäuseteil beziehungsweise den Kartuschenhalter vorgesehen.

### Beschreibung eines Ausführungsbeispiels

Weitere Ziele, Vorteile, Merkmale und Anwendungsmöglichkeiten der vorliegenden Erfindung ergeben sich aus der nachfolgenden Beschreibung eines Ausführungsbeispieles anhand der Zeichnungen. Dabei bilden alle beschriebenen und/oder bildlich dargestellten Merkmale für sich oder in beliebiger sinnvoller Kombination den Gegenstand der vorliegenden Erfindung, auch unabhängig von ihrer Zusammenfassung in den Ansprüchen und deren Rückbeziehung.

Es zeigen:
- Figur 1:: ein als Injektionsstift mit Schutzkappe ausgebildetes Ausführungsbeispiel einer erfindungsgemäßen Vorrichtung in einer perspektivischen Darstellung,
- Figur 1a:: der Injektionsstift gemäß Figur 1 ohne Schutzkappe mit aufgesetztem Kanülenhalter, auf dem eine Schutzkappe angeordnet ist.
- Figur 2:: der Injektionsstift gemäß Figur 1a ohne Schutzkappe für den Kanülenhalter,
- Figur 3:: eine Explosionsdarstellung des Injektionsstiftes gemäß Figur 1,
- Figur 4:: der Injektionsstift gemäß Figur 2 in einer Teilschnittsdarstellung,
- Figur 4a:: eine Detaildarstellung der Figur 4 in einer Schnittdarstellung,
- Figur 5:: eine perspektivische Ansicht des Injektionsstiftes in einer Momentaufnahme während des Lösen des Kanülenhalters von der Kanülenaufnahme in einer Teilschnittdarstellung,
- Figur 5a:: eine Detaildarstellung der Figur 5 in einer Schnittdarstellung,
- Figur 6:: eine perspektivische Ansicht des Injektionsstiftes in einer Momentaufnahme nach des Lösen des Kanülenhalters von der Kanülenaufnahme in einer Teilschnittdarstellung und
- Figur 6a:: eine Detaildarstellung der Figur 6 in einer Schnittdarstellung,

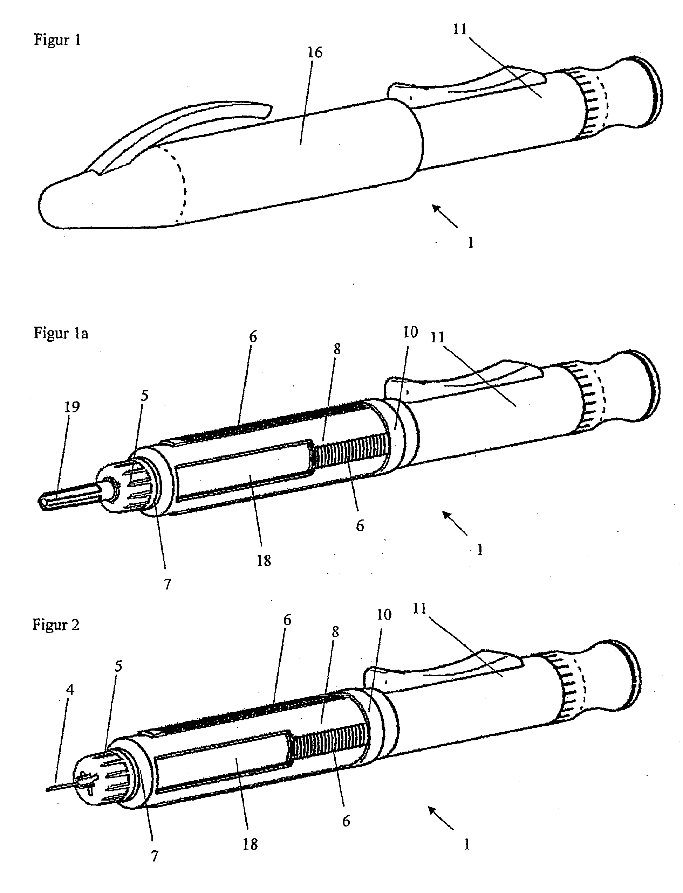

In Figur 1 ist ein als Injektionsstift ausgebildetes Ausführungsbeispiel einer erfindungsgemäßen Vorrichtung dargestellt. Der Injektionsstift besteht im Wesentlichen aus einem stiftförmigen Gehäuse 1, welches vorliegend aus einem Kartuschenhalter 10 und einer damit über ein Gewinde lösbar verbundenen Dosiereinrichtung 11 gebildet ist, wie dies insbesondere aus Figur 2 hervorgeht. In Figur 1 ist der Kartuschenhalter 10 durch eine Schutzkappe 16 abgedeckt. Der Kartuschenhalter 10 ist dabei zur Aufnahme einer ein flüssiges Medikament beinhaltenden Kartusche 2 ausgebildet, welche an ihrem durchstechbar abgedichteten, proximalen Ende eine Kanülenaufnahme 3 aufweist. An die Kanülenaufnahme 3 ist eine Kanülenhalter 5 lösbar anordnenbar. Der Kanülenhalter 5 trägt dabei eine Kanüle 4, welche durch das durchstechbar abgedichtete, proximale Ende der Kartusche 2 in deren Inneres hineinreicht, wenn die der Kanülenhalter 5 an der Kanülenaufnahme 3 angeordnet ist, wie dies ebenfalls in den Figuren 1a und 2 dargestellt ist. In Figur 1a ist die Kanüle 2 des Kanülenhalter 5 ebenfalls durch eine eigene Schutzkappe 19 abgedeckt.

An der Kanülenaufnahme 3 sind Kupplungsmittel 12 in Form von Federelementen 14 angeordnet, welche mit als Aussparungen 15 ausgebildeten Gegenkupplungsmittel 13 des Kanülenhalters 5 zusammenwirken. Dabei weisen die Federelemente 14 Vorsprünge 17 auf, welche im auf der Kanülenaufnahme 3 angeordneten Kanülenhalter 5 in dessen Aussparungen 15 eingreifen und so einen sicheren Sitz des Kanülenhalters 5 auf der Kanülenaufnahme 3 während des Injizierens des Medikamentes gewährleistet.

Auf dem Kartuschenhalter 10 ist längsverschiebbar ein Hülse 8 angeordnet. die Hülse 8 besteht im Wesentlichen aus einem Betätigungselement 6 und einem Löseelement 7, welche vorliegend einstückig ausgebildet sind. Zum Verschieben der Hülse 8 auf der Kartuschenhalterung 10 ist ein Führung der Hülse 8 vorgesehen. Das Löseelement 7 ist in dem beschriebenen Ausführungsbeispiel ringförmig ausgebildet und hintergreift den Kanülenhalter5, wenn dieser auf der Kanülenaufnahme 3 aufgesetzt ist.

Um den Füllstand des flüssigen Medikamentes innerhalb der Kartusche 2 kontinuierlich kontrollieren zu können, besteht die Kartusche 2 aus transparentem Material, wobei sowohl die Kartuschenhalterung 10 als auch die Hülse 8 weisen Sichtfenster 18 aufweisen. Dadurch ist zu jeder Zeit ein freier Blick auf den Kartuscheninhalt gewährleistet. Zu diesem Zweck wäre es natürlich auch möglich, sowohl die Kartuschenhalterung 10 als auch die Hülse 8 zumindest teilweise transparent auszugestalten.

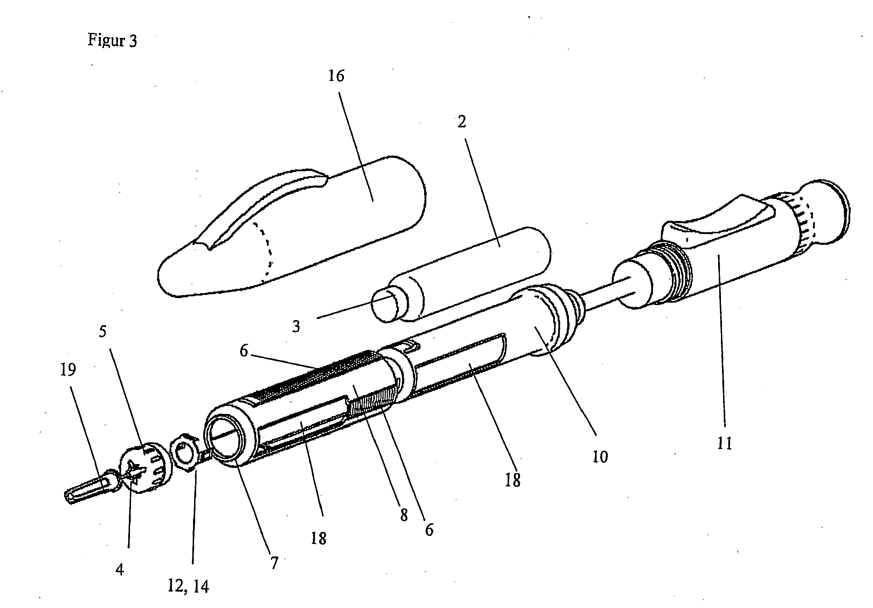

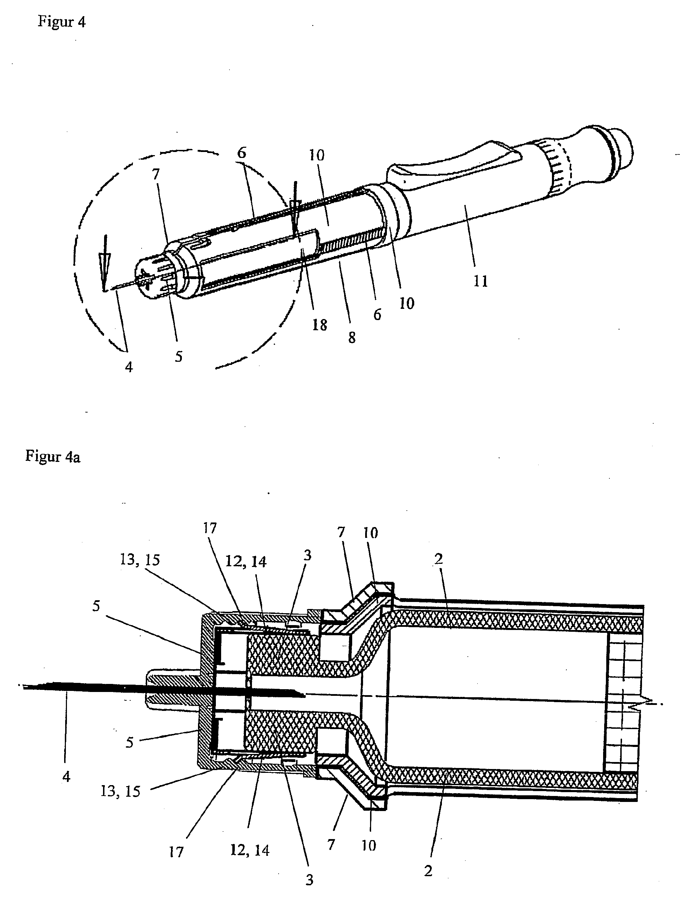

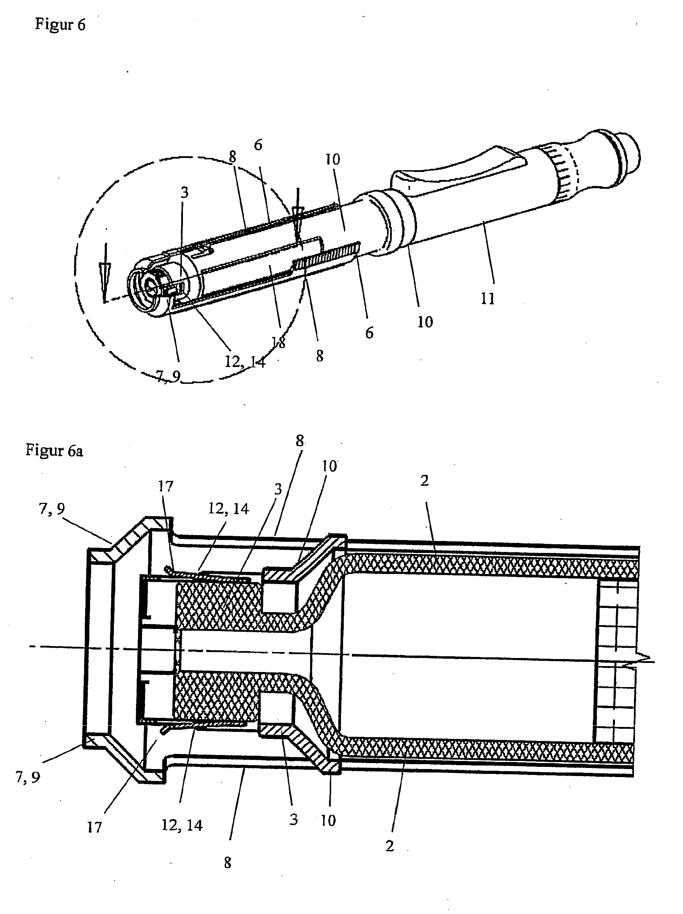

Die wesentlichen Einzelteile des Injektionsstiftes sind in der Figur 3 als Explosionsdarstellung dargestellt, während der funktionelle Zusammenhang der Einzelteile in den Schnittdarstellungen der Figuren 4 und 6 wiedergegeben ist.. In den Figuren 4, 5 und 6 wird der Injektionsstift in einer perspektivischen Ansicht in verschiedenen Momentaufnahmen während des Lösens des Kanülenhalters 5 von der Kanülenaufnahme 3 gezeigt. Dabei sind die Hülse 8 und der Kanülenhalter 5 zu einem Viertel ausgeschnitten, um eine Draufsicht auf die Kanülenaufnahme in diesen Momentaufnahmen zu gewährleisten. Die mit dem Zusatz "a" versehen Figuren stellen dabei eine Schnittdarstellung des Injektionsstiftes im Bereich der Kanülenaufnahme 3 dar.

In Figur 4 ist der Injektionsstift gemäß Figur 2 in einer perspektivischen Ansicht mit einer Teilschnittdarstellung der Hülse 8 und des Kanülenhalters 5 dargestellt. Wie bereits erwähnt, ist die Hülse 8 in einer Führung in axialer Richtung des Injektionsstiftes verschiebbar, wobei er in den Figuren 6 und 6a eine Endposition einnimmt, in welcher der Kanülenhalter 5 sicher auf der Kanülenaufnahme 2 sitzt. Der sichere Sitz wird dabei dadurch gewährleistet, dass ein an dem Federelement 14 angeordneter Vorsprung 17 in eine Aussparung 15 des Kanülenhalters5 eingreift.

In der in den Figuren 4 und 4a dargestellten Stellung ist der Injektionsstift zur Injektion einer mittels der Dosiereinrichtung 11 eingestellten Dosis des Medikamentes bereit. Nach der Injektion weisen die Hülse 8, der Kanülenhalters 5 und die Federelemente 14 immer noch die in den Figuren 4 und 4a dargestellte Position auf. Allerdings ist es nach der Injektion der durch die Dosiereinrichtung 11 vorgegebenen Dosis notwendig, die benutzte Kanüle 2 zu entsorgen. Dies geschieht, indem der Kanülenhalter 5 samt Kanüle 2, welche zusammen als Einwegeinheit ausgebildet sind, von der Kanülenaufnahme 3 und durch einen neuen ersetzt wird.

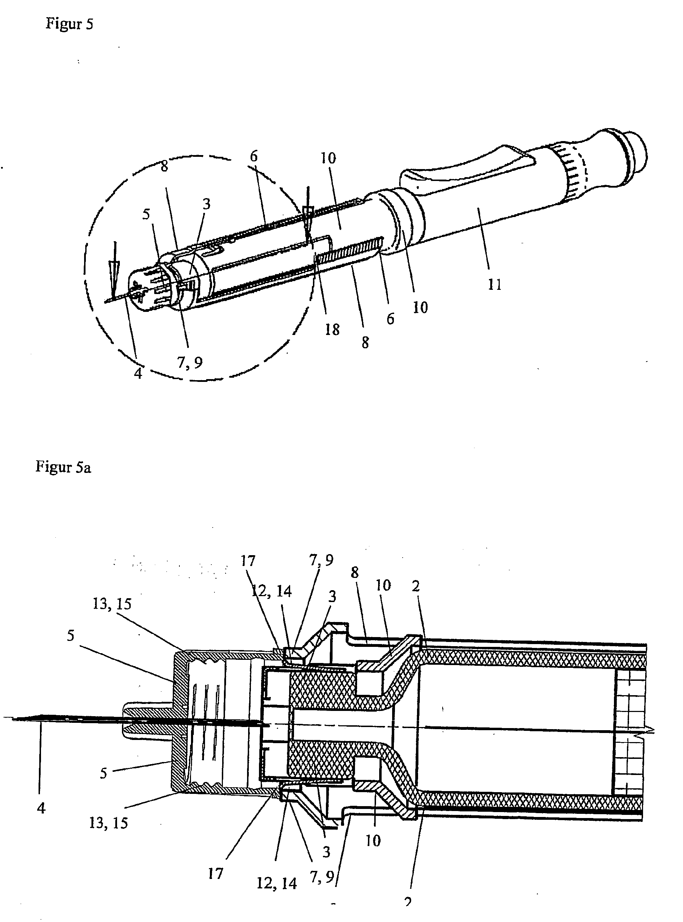

Im beschriebenen Ausfürungsbeispiel wird dazu das an der Hülse 8 angeordnete Betätigungselement 6 betätig. Mittels des Betätigungselementes 6 wird die gesamte Hülse 8 in Richtung des Kanülenhalters 5 in axialer Richtung des Injektionsstiftes verschoben. Dabei hintergreift das an der Hülse 8 angeordnete, ringförmige Löseelement 7 den Kanülenhalter 5 und schiebt ihn von der Kanülenaufnahme. Das axiale Verschieben der Hülse 8 kann dabei über eine in axialer Richtung auf dem Kartuschenhalter 10 angeordneten linearen Führung geschehen. Es ist allerdings auch möglich, dass ein Innengewinde der Hülse 8 mit einem Außengewinde des Kartuschenhalters 10 zusammenwirkt, wodurch eine Führung der Hülse 8 und somit ihr Verschieben in axialer Richtung des Injektionsstiftes ermöglicht ist. In den Figuren 5 und 5a ist eine Momentaufnahme während des Verschiebens der Hülse dargestellt. Insbesondere aus Figur 5a geht hervor, wie der an dem Federelement angeordnete Vorsprung 17 außer Eingriff mit der Aussparung 15 gerät und somit das Lösen des Kanülenhalters von der Kanülenaufnahme gestartet wird.

In der Führung für die Hülse 8 sind Anschläge vorgesehen, die ein gänzliches Lösen der Hülse 8 von der Kartuschenhalterung 10 verhindern. Die Positionen der Hülse 6 an diesen Anschlägen entsprechen den in den Figuren 4 und 6 beziehungsweise 4a und 4a dargestellten Positionen. In der Position gemäß den Figuren 8 und 8a ist die Hülse 8 bereits bis zu dem Anschlag verschoben, an dem das Löseelement 7 den Kanülenhalter 5 ganz von der Kanülenaufnahme geschoben hat. Der Kanülenhalter 8 ist nunmehr sicher von der Kanülenaufnahme 3 gelöst und in einem Entsorgungsbehälter, der während des Lösevorgangs bereitsteht entsorgt.

Die Hülse 8 kann nach dem Lösen manuell in die Ausgangsposition geführt werde. Alternativ ist es natürlich möglich, dass ein automatisierter Rückholmechanismus, beispielsweise in Form einer Rückholfeder, in den Injektionsstift integriert ist.

Ein neuer, unbenutzter Kanülenhalter 5 kann nun in gewohnter Weise auf der Kanülenaufnahme 3 angeordnet werden. Natürlich ist es auch möglich und aus hygienischen Gründen sinnvoll, die Schutzkappe 16 auf den Injektionsstift aufzusetzen und den neuen Kanülenhalter 10 erst kurz vor einer erneuten Injektion auf der Kanülenaufnahme 3 anzuordnen.

### Bezugszeichenliste

- 1 -: Gehäuseteil
- 2 -: Kartusche
- 3 -: Kanülenaufnahme
- 4 -: Kanüle
- 5 -: Kanülenhalter
- 6 -: Betätigungselement
- 7 -: Löseelement
- 8 -: Hülse
- 9 -: Begrenzung
- 10 -: Kartuschenhalter
- 11 -: Dosiereinrichtung
- 12 -: Kupplungsmittel
- 13 -: Gegenkupplungsmittel
- 14 -: Federelement
- 15 -: Aussparung
- 16 -: Schutzkappe
- 17 -: Vorsprung
- 18 -: Sichtfenster
- 19 -: Schutzkappe

## Patentansprüche

1. Vorrichtung zur Injektion von Flüssigkeiten, insbesondere von flüssigen Medikamenten beziehungsweise Hormonpräparaten wie beispielsweise Insulin, mit einem Gehäuseteil (1),
in oder an welchem eine die Flüssigkeit enthaltende Kartusche (2) anordnenbar ist,
wobei das Gehäuseteil (1) oder die Kartusche (2) eine Kanülenaufnahme (3) aufweist,
welche zum lösbaren Anordnen eines eine Kanüle (4) tragenden Kanülenhalters (5) ausgebildet ist und
wobei die Kartusche (2) beim Lösen des Kanülenhalters (5) von der Kanülenaufnahme (3) ortsfest in oder an dem Gehäuseteil (1) gehalten ist, wobei
zum Lösen des Kanülenhalters (5) ein an dem Kanülenhalter (5) angreifendes und durch ein Betätigungselement (6) aktivierbares Löseelement (7) vorgesehen ist und wobei das Löseelement (7) in axialer Richtung verschiebbar an oder auf dem Gehäuseteil (1) angeordnet ist und wobei das Löseelement (7) und/oder das Betätigungselement (6) entlang einer Führung an oder auf dem Gehäuseteil (1) verschiebbar angeordnet ist/sind,
**dadurch gekennzeichnet, dass**
die Führung als wenigstens eine lineare Führungsschiene in axialer Richtung des Gehäuseteils (1) ausgebildet ist, wobei an das Löseelement (7) und/oder das Betätigungselement (6) Elemente zum Eingriff in die wenigstens eine Führungsschiene angeordnet sind.

2. Vorrichtung nach Anspruch 1, **dadurch gekennzeichnet, dass** sie zum manuellen Lösen des Kanülenhalters (5) ausgebildet ist.

3. Vorrichtung nach Anspruch 1, **dadurch gekennzeichnet, dass** sie zum motorischen, insbesondere elektromotorischen Lösen des Kanülenhalters (5) ausgebildet ist.

4. Vorrichtung nach einem der vorhergehenden Ansprüche, **dadurch gekennzeichnet, dass** das Gehäuseteil (1) im Wesentlichen stiftförmig beziehungsweise axialsymmetrisch bezüglich seiner Längsachse ausgebildet ist.

5. Vorrichtung nach einem der vorhergehenden Ansprüche, **dadurch gekennzeichnet, dass** sie als Injektionsstift ausgebildet ist.

6. Vorrichtung nach einem der vorhergehenden Ansprüche, **dadurch gekennzeichnet, dass** das Löseelement (7) und das Betätigungselement (6) starr oder über Getriebemittel miteinander verbunden sind.

7. Vorrichtung nach einem der vorhergehenden Ansprüche, **dadurch gekennzeichnet, dass** als Führung ein Außengewinde an dem Gehäuseteil (1) angeordnet ist, welches mit einem Innengewinde des Löseelementes (7) und/oder des Betätigungselementes (6) zusammen wirkt.

8. Vorrichtung nach einem der vorhergehenden Ansprüche, **dadurch gekennzeichnet, dass** als Löseelement (7) und/oder als Betätigungselement (6) eine auf dem Gehäuseteil (1) axial verschiebbare Hülse (8) vorgesehen ist.

9. Vorrichtung nach einem der vorhergehenden Ansprüche, **dadurch gekennzeichnet, dass** das Löseelement (7) eine ringförmige Begrenzung (9) aufweist, die in Gebrauchstellung den auf der Kanülenaufnahme (3) angeordneten Kanülenhalter (5) hintergreift.

10. Vorrichtung nach einem der vorhergehenden Ansprüche, **dadurch gekennzeichnet, dass** das Löseelement (7) und/oder das Betätigungselement (6) durch wenigstens ein Fixierungselement fixierbar sind, um ein unbeabsichtigtes Lösen des Kanülenhalters (5) vom Kartuschenhalter zu verhindern.

11. Vorrichtung nach einem der vorhergehenden Ansprüche, **dadurch gekennzeichnet, dass** das Gehäuseteil (1) oder die Kartusche (2) Kupplungsmittel (12) aufweisen, welche mit entsprechenden Gegenkupplungsmittel (13) des Kanülenhalters korrespondieren.

12. Vorrichtung nach Anspruch 11, **dadurch gekennzeichnet, dass** die Kupplungsmittel (12) als Federelemente (14) und die Gegenkupplungsmittel (13) als damit korrespondierende Aussparungen (15), in welche die Federelemente (14) eingreifen, ausgebildet sind.

13. Vorrichtung nach Anspruch 11, **dadurch gekennzeichnet, dass** die Kupplungsmittel (12) und die Gegenkupplungsmittel (13) als Rast- und Gegenrastelemente beziehungsweise als Clips- und Gegenclipselemente ausgebildet sind.

14. Vorrichtung nach Anspruch 11, **dadurch gekennzeichnet, dass** die Kupplungsmittel (12) und die Gegenkupplungsmittel (13) als Außen- und Innengewinde ausgebildet sind.

## Claims

1. Device for the injection of fluids, in particular fluid medications, respectively hormone drugs such as insulin, having a casement (1),
in which or at which a fluid-containing cartridge (2) is disposed,
wherein the casement (1) or the cartridge (2) has a cannula mounting (3),
which is designed for detachable disposal of a cannula holder (5) equipped with a cannula (4) and
wherein the cartridge (2) is held stationary or at the casement (1) by the cannula mounting (3) upon detaching the cannula holder (5),
wherein
a detachment element (7) for detaching the cannula holder (5) is provided that engages at the cannula holder (5) and is activated by an operating element (6) and wherein the detachment element (7) is disposed in a slidable manner in axial direction at or on the casement (1) and wherein the detachment element (7) and/or the operating element (6) is/are disposed in a slidable manner along a guiding at or on the casement (1),
**characterized in that**
the guiding is realized as at least one guiding rail in axial direction of the casement (1), wherein elements for engaging in the at least one guiding rail are disposed at the detachment element (7) and/or the operating element (6).

2. Device according to claim 1, **characterized in that** it is designed for manual detachment of the cannula holder (5).

3. Device according to claim 1, **characterized in that** it is designed for motor, in particular electromotor detachment of the cannula holder (5).

4. Device according to one of the preceding claims, **characterized in that** the casement (1) is realized in a substantially pen-shaped, respectively axisymmetric manner in regard of its longitudinal axis.

5. Device according to one of the preceding claims, **characterized in that** it is designed as an injection pen.

6. Device according to one of the preceding claims, **characterized in that** the detachment element (7) and the operating element (6) are joined together in a rigid manner or via gear means.

7. Device according to one of the preceding claims, **characterized in that** an external thread is disposed at the casement (1) as a guiding that interacts with an internal thread of the detachment element (7) and/or the operating element (6).

8. Device according to one of the preceding claims, **characterized in that** a sleeve (8) that is axially moveable on the casement (1) is provided as detachment element (7) and/or operating element (6).

9. Device according to one of the preceding claims, **characterized in that** the detachment element (7) has an annular margin (9) that in its use position catches behind the cannula holder (5) disposed on the cannula mounting (3).

10. Device according to one of the preceding claims, **characterized in that** the detachment element (7) and/or the operating element (6) are fixable by at least one fixation element for impeding an unintended detachment of the cannula holder (5) from the cartridge holder.

11. Device according to one of the preceding claims, **characterized in that** the casement (1) or the cartridge (2) have coupling means (12) that correspond to the counter coupling means (13) of the cannula holder.

12. Device according to claim 11, **characterized in that** the coupling means (12) are realized as spring elements (14) and the counter coupling means (13) as corresponding recesses (15) in which the spring elements (14) engage.

13. Device according to claim 11, **characterized in that** the coupling means (12) and the counter coupling means (13) are realized as counter snap elements or as clips and counter clip elements.

14. Device according to claim 11, **characterized in that** the coupling means (12) and the counter coupling means (13) are realized as external thread and internal thread.

## Revendications

1. Dispositif pour l'injection de fluides, particulièrement de médicaments fluides, respectivement de préparations hormonales comme l'insuline, avec une caisse (1) dans laquelle ou à laquelle une cartouche (2) contenant de fluide peut être arrangée,
dans lequel la caisse (1) ou la cartouche (2) ont une monture de canule (3),
qui est désignée pour le montage détachable d'un support de canule (5) portant une canule (4), et
dans lequel la cartouche (2) est tenue stationnairement ou à la caisse (1) par la monture de canule (3) quand le support de canule (5) est détaché,
dans lequel
un élément de détachement (7) pour détacher le support de canule (5) est pourvu qui s'engrène dans le support de canule (5) et est activé par un élément d'actionnement (6) et dans lequel l'élément de détachement (7) est disposé de manière glissante en direction axiale à ou sur la caisse (1) et dans lequel l'élément de détachement (7) et/ou l'élément d'actionnement (6) est/sont arrangé(s) de manière glissante le long d'un guidage à ou sur la caisse (1),
**caractérisé en ce que**
le guidage est réalisé comme au moins un rail de guidage en direction axiale de la caisse (1), dans lequel des éléments pour s'engrener dans le au moins un rail de guidage sont placés à l'élément de détachement (7) et/ou à l'élément d'actionnement (6).

2. Dispositif selon la revendication 1, **caractérisé en ce qu'**il est désigné pour un détachement manuel du support de canule (5).

3. Dispositif selon la revendication 1, **caractérisé en ce qu'**il est désigné pour un détachement par moteur, particulièrement par un électromoteur du support de canule (5).

4. Dispositif selon une des revendications précédentes, **caractérisé en ce que** la caisse (1) est réalisée substantiellement styliforme, respectivement axisymétrique en égard de son axe longitudinal.

5. Dispositif selon une des revendications précédentes, **caractérisé en ce qu'**il est désigné comme stylo injecteur.

6. Dispositif selon une des revendications précédentes, **caractérisé en ce que** l'élément de détachement (7) et/ou l'élément d'actionnement (6) sont associés rigidement ou par des moyens d'engrenage.

7. Dispositif selon une des revendications précédentes, **caractérisé en ce qu'**un filet extérieur est arrangé à la caisse (1) comme guidage qui interagit avec un filet intérieur de l'élément de détachement (7) et/ou de l'élément d'actionnement (6).

8. Dispositif selon une des revendications précédentes, **caractérisé en ce qu'**une manche (8) axialement déplaçable sur la caisse (1) est pourvue comme élément de détachement (7) et/ou élément d'actionnement (6).

9. Dispositif selon une des revendications précédentes, **caractérisé en ce que** l'élément de détachement (7) a une marge annulaire (9) qui dans sa position d'utilisation prend derrière le support de canule (5) arrangé sur la monture de canule (3).

10. Dispositif selon une des revendications précédentes, **caractérisé en ce que** l'élément de détachement (7) et l'élément d'actionnement (6) sont fixables par au moins un élément de fixation pour empêcher un détachement involontaire du support de canule (5) du support de cartouche.

11. Dispositif selon une des revendications précédentes, **caractérisé en ce que** la caisse (1) ou la cartouche (2) ont des moyens de couplage (12) correspondants aux moyens de couplage opposés (13) du support de canule.

12. Dispositif selon la revendication 11, **caractérisé en ce que** les moyens de couplage (12) sont réalisés comme des éléments de ressort (14) et les moyens de couplage opposés (13) comme des échancrures (15) correspondantes dans lesquelles les éléments de ressort (14) s'engrènent.

13. Dispositif selon la revendication 11, **caractérisé en ce que** les moyens de couplage (12) et les moyens de couplage opposés (13) sont réalisés comme des éléments d'enclenchement ou comme des éléments de clip et clip opposé.

14. Dispositif selon la revendication 11, **caractérisé en ce que** les moyens de couplage (12) et les moyens de couplage opposés (13) sont réalisés comme filet extérieur et filet intérieur.
